# EUROPEAN PATENT SPECIFICATION

(11) **EP 1 587 553 B1**
(45) Date of publication and mention of the grant of the patent: **11.03.2009**
(21) Application number: 03796608.2
(22) Date of filing: 03.12.2003
(51) Int. Cl.: A61L 12/14, A61K 31/16

(54) **QUATERNARY AMMONIUM ESTERS FOR DISINFECTION AND PRESERVATION**
QUATERNÄRE AMMONIUMESTER ZUR DESINFEKTION UND AUFBEWAHRUNG
ESTERS D'AMMONIUM QUATERNAIRE POUR LA DESINFECTION ET LA CONSERVATION

(30) Priority: 13.12.2002 US 433624 P; 11.04.2003 US 412796
(43) Date of publication of application: 26.10.2005
(73) Proprietor: BAUSCH & LOMB INCORPORATED, Rochester, New York 14604-2701 (US)
(72) Inventor: SALAMONE, Joseph, C., Fairport, NY 14450 (US); OZARK, Richard, Solvay, NY 13209 (US); HU, Zhenze, Pittsford, NY 14534 (US); BORAZJANI, Roya, Nicole, Fairport, NY 14450 (US)
(74) Representative: Maiwald, Walter
(86) International application number: PCT/US2003/038434
(87) International publication number: WO 2004/054630

(56) References cited:
- US-A- 3 910 971
- US-A- 4 255 273
- US-A- 5 631 005

## Description

### Field of the Invention:

The present invention is directed toward the use of quaternary ammonium esters for disinfection and preservation. More particularly, the present invention is directed toward the use of quaternary ammonium esters for disinfection and preservation of ophthalmic solutions and devices.

### Background of the Invention:

Contact lenses in wide use today fall into two general categories, hard and soft. The hard or rigid corneal type lenses are formed from materials prepared by the polymerization of acrylic esters, such as poly(methyl methacrylate) (PMMA). The gel, hydrogel or soft type lenses are made by polymerizing such monomers as 2-hydroxyethyl methacrylate (HEMA) or, in the case of extended wear lenses, by polymerizing siloxy-containing monomers or macromonomers. Both the hard and soft types of contact lenses are exposed to a broad spectrum of microbes during normal wear and become soiled relatively quickly. Contact lenses whether hard or soft therefore require routine cleaning and disinfecting. Failure to routinely clean and disinfect contact lenses properly can lead to a variety of problems ranging from mere discomfort when being worn to serious ocular infections. Ocular infections caused by virulent microbes such as *Pseudomonas aeruginosa* can lead to loss of the infected eye(s) if left untreated or if allowed to reach an advanced stage before initiating treatment.

Dassanayake et al. disclose in U.S. Patent Number 5,631,005 the use of certain amidoamines to disinfect contact lenses and to preserve ophthalmic compositions. When such amidoamines are quaternized, the disinfection ability thereof is greatly reduced. For example, the composition N-myristamidopropyl-N,N-dimethylamine, represented by the structure 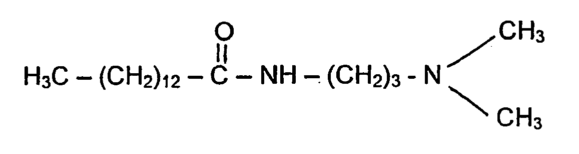 was effective against *Staphylococcus aureus, Pseudomonas aeruginosa, Candida albicans* and *Fusarium solani* but was not effective against *Serratia marcescens*. This data was obtained in BBS at 25 ppm concentration. When like data was obtained on the analogous ester of the above structure, N-myristoyloxypropyl-N,B-dimethylamine, as represented by the structure below, 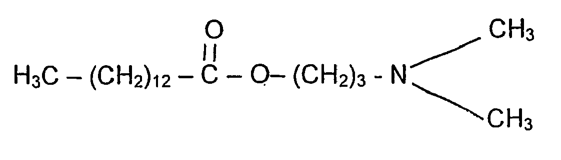 the same failed to be effective against each of the five microorganisms studied, i.e., *S. aureus, P. aeruguinosa, C. albicans, F. solani* and *S. marcescens*. Likewise, when similar stand-alone biocidal data was obtained, the quaternary composition N-myristamidopropyl-N,N,N-trimethylammonium iodide, represented by the structure 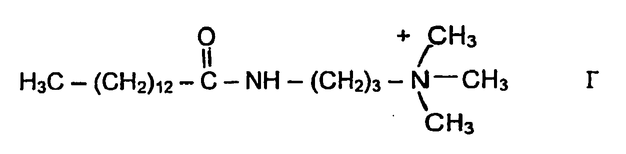 proved effective against *S. aureus* and *P. aeruginosa* but failed for S. *marcescens, C. albicans* and *F. solani*. Thus, the disinfection ability of the amidoamine compound, Aldox^{™} (Alcon Laboratories, Inc., Fort Worth, Texas), used commercially to enhance disinfection of fungi, is greatly diminished when quaternized.

US 3,910,971 describes certain quaternary ammonium ester compounds as bacteriostats. EP 0 937 771 A1 describes a liquid detergent composition comprising a diester or diamide quaternary ammonium compound.

Despite the availability of various commercially available contact lens disinfecting systems such as heat, hydrogen peroxide, amidoamines and other chemical agents, there continues to be a need for improved disinfecting systems. Such improved disinfecting systems include systems that are simple to use, are effective against a broad spectrum of microbes, are non-toxic and do not cause ocular irritation. There is a particular need in the field of contact lens disinfection and ophthalmic composition preservation for safe and effective chemical agents with antimicrobial activity.

### Summary of the Invention:

The present invention relates to ophthalmic solutions and the use of solutions as defined in the claims.

The quaternary ammonium esters of the present invention are generally represented by the structure of Formula 1 below 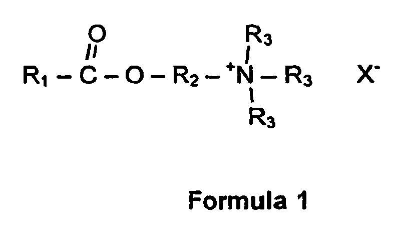 wherein R₁ is a saturated or unsaturated and branched or unbranched C₁₋₂₄ alkyl, C₁₋₂₄ alkene, C₆₋₂₄ aryl, C₆₋₃₆ arylalkyl, C₁₋₂₄ alkyloxy, C₁₋₂₄ haloalkyl or C₆₋₂₄ haloaryl; R₂ is branched or unbranched C₁₋₂₄ alkylene, C₆₋₃₆ alkylenearylene, C₆₋₂₄ arylene, C₁₋₂₄ alkyloxy or C₆₋₃₆ aryloxy; the R₃ groups may be the same or different saturated or unsaturated and branched or unbranched C₁₋₂₄ alkyl, C₆₋₃₆ arylalkyl, C₁₋₂₄ hydroxyalkyl, C₁₋₂₄ alkoxyalkyl, C₁₋₂₄ alkoxyalkoxyalkyl or C₁₋₂₄ haloalkyl; and X is a halide, a C₁₋₂₄ alkylsulfate, a C₆₋₃₆ arylsulfate, or other pharmaceutically acceptable salt.

The subject quaternary ammonium esters are effective antimicrobial agents useful in the manufacture of disinfecting systems that are non-toxic, simple to use and do not cause ocular irritation.

Accordingly, it is an object of the present invention to provide solutions useful as antimicrobial agents in the manufacture of ophthalmic disinfecting systems.

Another object of the present invention is to provide solutions useful in ophthalmic systems for disinfecting contact lenses.

Another object of the present invention is to provide solutions useful in preserving ophthalmic systems from microbial contamination.

Another object of the present invention is to provide solutions useful in ophthalmic systems for disinfecting contact lenses with reduced or eliminated eye irritation.

Another object of the present invention is to provide enhanced surface activity and, hence, cleaning.

Still another object of the present invention is to provide a method of making solutions useful as antimicrobial agents.

These and other objectives and advantages of the present invention, some of which are specifically described and others that are not, will become apparent from the detailed description and claims that follow.

### Detailed Description of the Invention:

The quaternary ammonium ester comprising solutions of the present invention can be used with all contact lenses such as conventional hard and soft lenses, as well as, rigid and soft gas permeable lenses. Such lenses include both hydrogel and non-hydrogel lenses, but the quaternary ammonium ester comprising solutions of the present invention are especially useful for soft lenses such as silicone and fluorine-containing lenses. Of primary interest are soft lenses fabricated from a material having a proportion of hydrophilic repeat units such that the water content of the lens during use is at least 20 percent by weight. The term "soft contact lens" as used herein generally refers to those contact lenses that readily flex under small amounts of force. Typically, soft contact lenses are formulated from polymers having a certain proportion of repeat units derived from monomers such as 2-hydroxyethyl methacrylate and/or other hydrophilic monomers, typically crosslinked with a crosslinking agent. However, newer soft lenses, especially for extended wear, are being made from high-Dk silicone-containing materials.

The quaternary ammonium ester compounds of the present invention are useful in contact lens care solutions for disinfecting contact lenses. In order to disinfect, a solution of the present invention must contain in deionized water one or more of the present quaternary ammonium ester compounds in sufficient concentrations to destroy harmful microorganisms on the surface of a contact lens within the recommended minimum soaking time. The recommended minimum soaking time is included in the package instructions for use of the solution. The term "disinfecting solution" does not exclude the possibility that the solution may also be useful as a preserving solution, or that the disinfecting solution may be useful for other purposes such as daily cleaning, rinsing, and storage of contact lenses, depending on the particular formulation containing the subject compositions. Additionally, the quaternary ammonium compounds of the present invention can be used in conjunction with other known disinfecting or preserving compounds if desired.

Solutions according to the present invention are physiologically compatible. Specifically, the solution must be "ophthalmically safe" for use with a contact lens, meaning that a contact lens treated with the solution is generally suitable and safe for direct placement on the eye without rinsing, that is, the solution is safe and comfortable for daily contact with the eye via a contact lens that has been wetted with the solution. An ophthalmically safe solution has a tonicity and pH that is compatible with the eye and comprises materials, and amounts thereof, that are non-cytotoxic according to ISO (International Standards Organization) standards and U.S. FDA (Food and Drug Administration) regulations. The solution should be sterile in that the absence of microbial contaminants in the product prior to release must be statistically demonstrated to the degree necessary for such products.

Solutions of the present invention include at least one quaternary ammonium ester compound of the present invention as generally represented by the structure of Formula 1 below 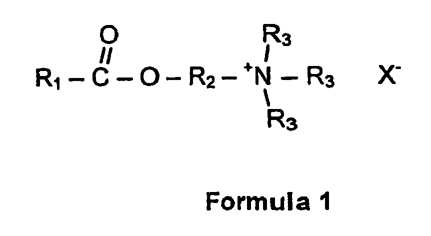 wherein R₁ is a saturated or unsaturated and branched or unbranched C₁₋₂₄ alkyl such as for example but not limited to methyl, propyl, hexyl or tridecyl, C₁₋₂₄ alkene such as for example but not limited to hexene or dodecene, C₆₋₂₄ aryl such as for example but not limited to phenyl, naphthyl or biphenyl, C₈₋₃₆ arylalkyl such as for example but not limited to alkyl-substituted phenyl groups or phenyl-substituted alkyl groups, C₁₋₂₄ alkyloxy such as for example but not limited to propoxy or butoxy, C₁₋₂₄ haloalkyl such as for example but not limited to fluoro-, chloro-, or bromo-substituted alkyl groups or C₆₋₂₄ haloaryl such as for example but not limited to chlorophenyl; R₂ is branched or unbranched C₁₋₂₄ alkylene such as for example but not limited to methylene, ethylene, propylene or butylene, C₆₋₃₆ alkylenearylene such as for example but not limited to methylenephenylene, C₆₋₂₄ arylene such as for example but not limited to phenylene, C₁₋₂₄ alkyloxy such as for example but not limited to methoxy, ethoxy or propoxy or C₆₋₃₆ aryloxy such as for example but not limited to phenoxy; the R₃ groups may be the same or different saturated or unsaturated and branched or unbranched C₁₋₂₄ alkyl such as for example but not limited to methyl or butyl, C₆₋₃₆ arylalkyl such as for example but not limited to alkyl-substituted phenyl groups or phenyl-substituted alkyl groups, C₁₋₂₄ hydroxyalkyl such as for example but not limited to hydroxyethyl or hydroxypropyl, C₁₋₂₄ alkoxyalkyl such as for example but not limited to ethoxyethyl or ethoxypropyl, C₁₋₂₄ alkoxyalkoxyalkyl such as for example but not limited to methoxyethoxyethyl or methoxyethoxypropyl or C₁₋₂₄ haloalkyl such as for example but not limited to fluoromethyl or chloropentyl; and X is a halide such as for example but not limited to chloride, bromide or iodide, a C₁₋₂₄ alkylsulfate, a C₆₋₃₆ arylsulfate or other pharmaceutically acceptable salt.

The subject quaternary ammonium ester compounds of the present invention can be synthesized in accordance with the reaction scheme illustrated in Scheme 1 below. 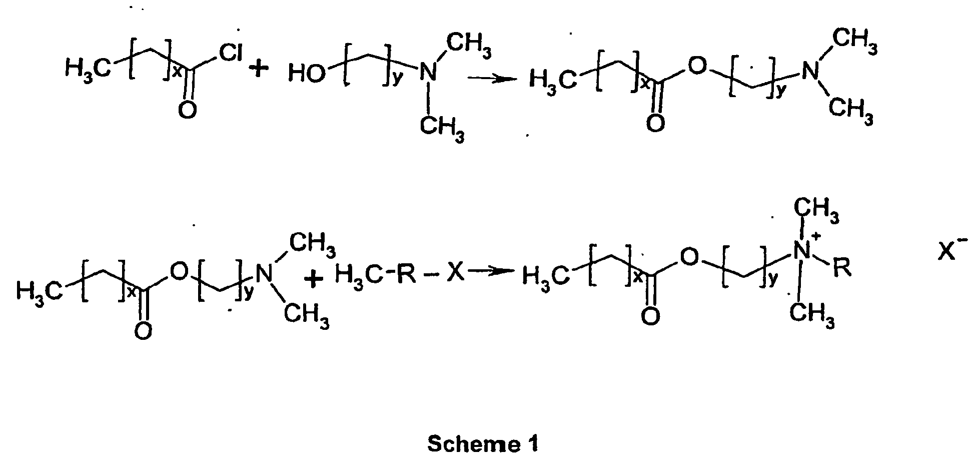 X⁻ = an anion such as for example but not limited to Cl⁻, Br⁻, I⁻ or CH₃SO₄⁻
R = an alkylating group

Quaternary ammonium esters of the present invention were produced in accordance with Scheme 1 above. The synthesis and biocidal study of the subject quaternary ammonium ester compounds are described in still greater detail in the examples that follow.

### Example I: Synthesis of Quaternary Ammonium Esters

### A. Synthesis of Amino Esters

A hydroxyamine (0.0932 mole) in 100 ml of chloroform was added dropwise to a cold, i.e., 0 °C, chloroform solution (300 ml) of acid chloride (0.0810 mole). After the addition was complete, the cold bath was removed and the reaction stirred overnight under nitrogen. A 400 ml saturated sodium bicarbonate solution was added and the mixture stirred for 45 minutes. The organic layer was washed with 200 ml of aqueous sodium bicarbonate/sodium chloride solution, dried over magnesium sulfate, filtered and concentrated in vacuum. The compounds were used the synthesis of quaternary ammonium esters without further purification.

### B. Synthesis of Quaternary Ammonium Esters

Five grams of the aminoester and the appropriate weight of quatemizing agent were dissolved in 50 ml of ethyl acetate. The solution was stirred overnight under nitrogen. The precipitate was filtered and dried overnight in a vacuum oven at 40 °C. The salt was recrystallized from ethyl acetate.

Specific examples of compounds of the present invention are set forth below in Table 1.

**Table 1**

| **Quaternary Ammonium Esters** | | |
|---|---|---|
| **Comp. No.** | **Name** | |
| 1. | N-myristoyloxypropyl-N-benzyl-N,N-dimethylammonium chloride | |
| | | Formula: C₂₆H₄₆NO₂Cl |
| | | m.p. (⁰C): 93-95 |
| | | Structure: |
| | | 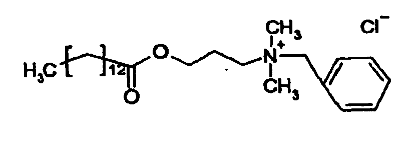 |
| 2. | N-capryloyloxypropyl-N,N,N-trimethylammonium iodide | |
| | | Formula: C₁₄H₃₀NO₂I |
| | | m.p. (⁰C): 107-109 |
| | | Structure: |
| | | 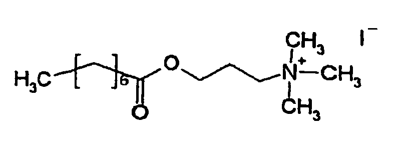 |
| 3. | N-caproyloxypropyl-N,N,N-trimethylammonium iodide | |
| | | Formula: C₁₆H₃₄NO₂I |
| | | m.p. (⁰C): 120-122 |
| | | Structure: |
| | | 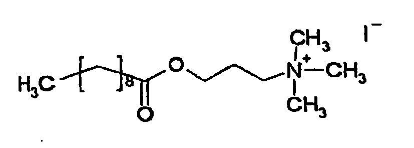 |
| 4. | N-lauroyloxypropyl-N,N,N-trimethylammonium iodide | |
| | | Formula: C₁₈H₃₈NO₂I |
| | | m.p. (⁰C): 121-124 |
| | | Structure: |
| | | 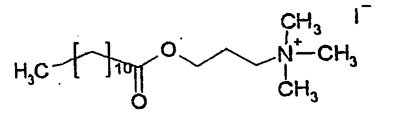 |
| 5. | N-myristoyloxypropyl-N,N,N-trimethylammonium iodide | |
| | | Formula: C₂₀H₄₂NO₂I |
| | | m.p. (⁰C): 118-120 |
| | | Structure: |
| | | 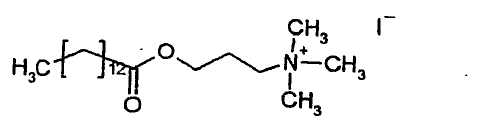 |
| 6. | N-palmitoyloxypropyl-N,N,N-trimethylammonium iodide | |
| | | Formula: C₂₂H₄₆NO₂I |
| | | m.p. (⁰C): 116-118 |
| | | Structure : |
| | | 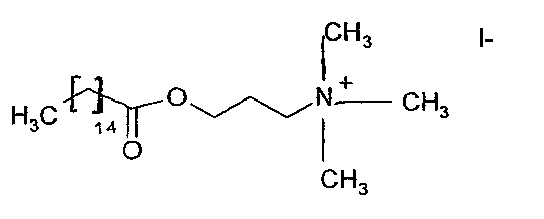 |
| 7. | N-stearoyloxypropyl-N,N,N-trimethylammonium iodide | |
| | | Formula: C₂₄H₅₀NO₂I |
| | | m.p. (⁰C): 116-118 |
| | | Structure: |
| | | 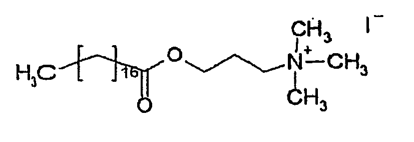 |
| 8. | N-myristoyloxypropyl-N,N-dimethyl-N-ethylammonium iodide | |
| | | Formula: C₂₁H₄₄NO₂I |
| | | m.p. (⁰C) 60-62 |
| | | Structure: |
| | | 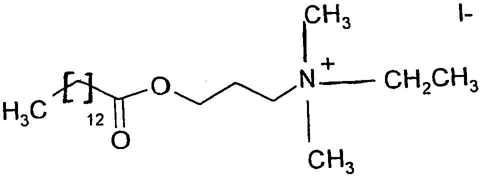 |
| 9. | N-myristoyloxypropyl-N,N-dimethyl-N-propylammonium iodide | |
| | | Formula: C₂₂H₄₆NO₂I |
| | | m.p. (⁰C): 80-82 |
| | | Structure: |
| | | 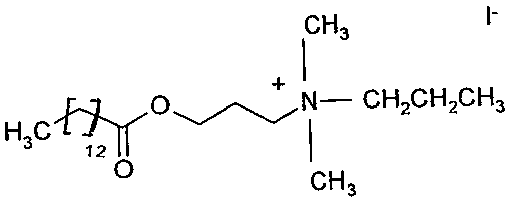 |
| 10. | N-myristoyloxypropyl-N-butyl-N,N-dimethylammonium iodide | |
| | | Formula: C₂₃H₄₈NO₂I |
| | | m.p. (⁰C): 87-89 |
| | | Structure: |
| | | 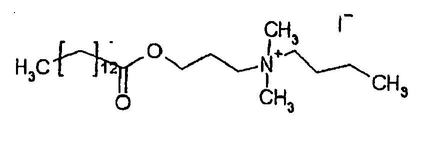 |
| 11. | N-myristoyloxypropyl-N,N-dimethyl-N-pentylammonium iodide | |
| | | Formula: C₂₄H₅₀NO₂I |
| | | m.p. (⁰C): 90-91 |
| | | Structure: |
| | | 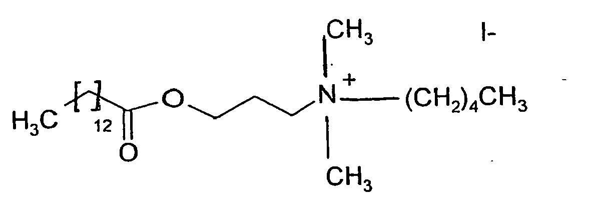 |
| 12. | N-myristoyloxypropyl-N,N-dimethyl-N-hexylammonium iodide | |
| | | Formula: C₂₅H₅₂NO₂I |
| | | m.p. (⁰C): 78-79 |
| | | Structure: |
| | | 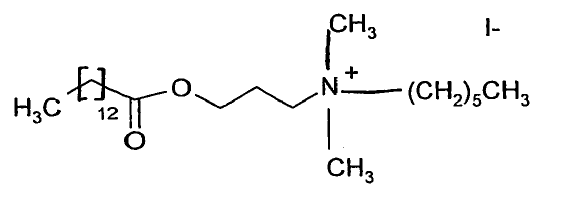 |
| 13. | N-myristoyloxypropyl-N-decyl-N,N-dimethylammonium iodide | |
| | | Formula: C₂₉H₆₀NO₂I |
| | | m.p. (⁰C): 103-104 |
| | | Structure: |
| | | 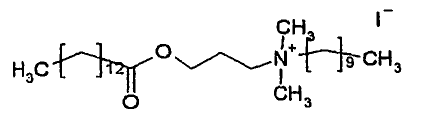 |
| 14. | N-myristoyloxyethyl-N,N-di(hydroxyethyl)-N-methylammonium iodide | |
| | | Formula: C₂₁H₄₄NO₄I |
| | | m.p. (⁰C): 48-50 |
| | | Structure: |
| | | 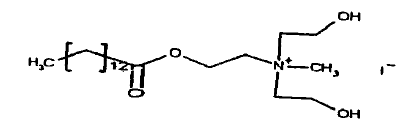 |
| 15. | N-myristoyloxyethoxyethyl-N,N,N-trirnethylammonium iodide | |
| | | Formula: C₂₁H₄₄NO₃I |
| | | m.p. (⁰C): 92-94 |
| | | Structure: |
| | | 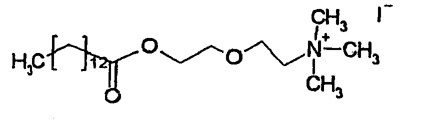 |

### Example II: Bactericidal and Fungicidal Study Procedure

A test was conducted to study the microbiocidal efficacy of a solution containing a quaternary ammonium ester of the present invention employing borate buffered saline (BBS). The antimicrobial efficacy of each of the various quaternary ammonium ester comprising solutions for the chemical disinfection of contact lenses was evaluated. Microbial challenge inoculums were prepared using *Pseudomonas aeruginosa* (ATCC 9027), *Staphylococcus aureus* (ATCC 6538), *Serratia marcescens* (ATCC 13880), *Candida albicans* (ATCC 10231) and *Fusarium solani* (ATCC 36031). The test organisms were cultured on appropriate agar and the cultures were harvested using sterile DPBST (Dulbecco's Phosphate Buffered Saline plus 0.05 percent weight/volume polysorbate 80) or a suitable diluent and transferred to a suitable vessel. Spore suspensions were filtered through sterile glass wool to remove hyphal fragments. *Serratia marcescens*, as appropriate, was filtered through a 1.2 mµ filter to clarify the suspension. After harvesting, the suspension was centrifuged at no more than 5000 x g for a maximum of 30 minutes at 20 to 25°C. The supernatant was poured off and resuspended in DPBST or other suitable diluent. The suspension was centrifuged a second time, and resuspended in DPBST or other suitable diluent. All challenge bacterial and fungal cell suspensions were adjusted with DPBST or other suitable diluent to 1 × 10 ⁷ to 1 × 10 ⁸ cfu/ml. The appropriate cell concentration may be estimated by measuring the turbidity of the suspension, for example, using a spectrophotometer at a preselected wavelength, for example 490 nm. One tube was prepared containing a minimum of 10 ml of test solution per challenge organism. Each tube of the solution to be tested was inoculated with a suspension of the test organism sufficient to provide a final count of 1 × 10⁵ to 1 × 10 ⁶ cfu/ml, the volume of the inoculum not exceeding 1 percent of the sample volume. Dispersion of the inoculum was ensured by vortexing the sample for at least 15 seconds. The inoculated product was stored at 10 to 25 °C. Aliquots in the amount of 1.0 ml were taken of the inoculated product for determination of viable counts after certain time periods of disinfection. The time points for the bacteria were, for example, 1, 2, 3 and 4 hours when the proposed regimen soaking time was four hours. Yeast and mold were tested at an additional timepoint of 16 hours (4 times the regimen time). The suspension was mixed well by vortexing vigorously for at least 5 seconds. The 1.0 ml aliquots removed at the specified time intervals were subjected to a suitable series of decimal dilutions in validated neutralizing media. The suspensions were mixed vigorously and incubated for a suitable period of time to allow for neutralization of the microbial agent at room temperature. The viable count of organisms was determined in appropriate dilutions by preparation of triplicate plates of trypticase soy (TSA) agar for bacteria and Sabouraud dextrose agar (SDA) for mold and yeast. The bacterial recovery plates were incubated at 30 to 35 °C for two to four days. The yeast was incubated at 20 to 30 °C for two to four days and mold recovery plates were incubated at 20 to 25°C for three to seven days. The average number of colony forming units was determined on countable plates. Countable plates refer to 30 to 300 cfu/plates for bacteria and yeast, and 8 to 80 cfu/plate for mold except when colonies are observed only for the 10⁰ or 10⁻¹ dilution plates. The microbial reduction was then calculated at the specified time points. In order to demonstrate the suitability of the medium used for growth of the test organisms and to provide an estimation of the initial inoculum concentration, inoculum controls were made by dispersing an identical aliquot of the inoculum into a suitable diluent, for example DPBST, using the same volume of diluent used to suspend the organism as listed above. Following inoculation in a validated neutralizing broth and incubation for an appropriate period of time, the inoculum control must be between 1.0 x 10⁵ to 1.0 x 10⁶ cfu/ml.

The solutions were evaluated based on the performance requirement referred to as the "Stand-Alone Procedure for Disinfecting Products" (hereafter the "stand-alone test") and is based on the Disinfection Efficacy Testing for contact lens care products under the Premarket Notification (510(k)) Guidance Document for Contact Lens Care Products dated May 1, 1997, prepared by the U.S. Food and Drug Administration, Division of Ophthalmic Devices. This performance requirement does not contain a rub procedure. This performance requirement is comparable to current ISO standards for disinfection of contact lenses (revised 1995). The stand-alone test challenges a disinfecting product with a standard inoculum of a representative range of microorganisms and establishes the extent of viability loss at predetermined time intervals comparable with those during which the product may be used. The primary criteria for a given disinfection period (corresponding to a potential minimum recommended disinfection period) is that the number of bacteria recovered per mL must be reduced by a mean value of not less than 3.0 logs within the given disinfection period. The number of mold and yeast recovered per ml must be reduced by a mean value of not less than 1.0 log within the minimum recommended disinfection time with no increase at four times the minimum recommended disinfection time.

The results of the subject microbiocidal study are set forth below in Table 2.

**Table 2**

| **Stand-alone Biocidal Results without Organic Soil (25 ppm in BBS)** | | | | | | |
|---|---|---|---|---|---|---|
| **Comp.No.** | **Time** | ***S.aureus*** | ***P.aeruginosa*** | ***S.marcescens*** | ***C.albicans*** | ***F.solani*** |
| 1 | 1 hr. | 4.6 | >4.7 | 4.8 | 3.6 | 4.0 |
| | 4 hr. | >4.8 | >4.7 | >4.8 | >4.8 | 4.2 |
| | | | | | | |
| 2 | 1 hr. | TNTC | TNTC | TNTC | 0.0 | 0.1 |
| | 4 hr. | TNTC | TNTC | TNTC | 0.2 | 0.6 |
| | | | | | | |
| 3 | 1 hr. | TNTC | TNTC | TNTC | 0.9 | 0.0 |
| | 4 hr. | TNTC | TNTC | TNTC | TNTC | 0.5 |
| | | | | | | |
| 4 | 1 hr. | TNTC | 2.0 | TNTC | 0.0 | 0.1 |
| | 4 hr. | TNTC | 3.0 | TNTC | 0.1 | 0.6 |
| | | | | | | |
| 5 | 1 hr. | >4.7 | >4.6 | 3.4 | 1.9 | 3.2 |
| | 4 hr. | >4.7 | >4.6 | >4.6 | 3.4 | 3.3 |
| | | | | | | |
| 7 | 1 hr. | 3.5 | 2.0 | TNTC | 0.7 | 2.9 |
| | 4 hr. | >4.8 | 3.3 | TNTC | 2.5 | 3.5 |
| | | | | | | |
| 10 | 1 hr. | >4.8 | >4.7 | 3.8 | 4.5 | >4.5 |
| | 4 hr. | >4.8 | 4.7 | >4.8 | >4.8 | >4.5 |
| | | | | | | |
| 13 | 1 hr. | 3.8 | 3.5 | TNTC | 0.5 | 1.5 |
| | 4 hr. | 4.8 | 4.7 | 2.9 | 1.2 | 3.1 |
| 14 | 1 hr. | 4.4 | 4.6 | 3.2 | 1.9 | 3.7 |
| | 4 hr. | >4.6 | >4.6 | 4.6 | 3.3 | 3.7 |
| | | | | | | |
| 15 | 1 hr. | >4.8 | >4.7 | 3.8 | 3.6 | 3.8 |
| | 4 hr. | >4.8 | >4.7 | >4.8 | 4.6 | 4.1 |

| | | | | | | |
|---|---|---|---|---|---|---|
| TNTC = Too numerous to count NT = Not tested | | | | | | |

### Example III: Amoebacidal Study Procedure

### A. Acanthamoeba polyphaga:

*Acanthamoeba polyphaga* strain Ros were used in this study. Trophozoites were maintained at 30°C in an axenic broth medium composed of 20.0 g Biosate^{™} (Becton Dickinson, United Kingdom), 5.0 g glucose, 0.3 g KH₂PO₄, 10 µg vitamin B₁₂ and 15.0 mg L-methionine per litre of deionized water. The pH was adjusted to 6.5 - 6.6 with 1 M NaOH before autoclaving at 121°C for 12 minutes. Penicillin and streptomycin were added to a final concentration of 150 µ/ml before use.

Cysts were prepared from trophozoite cultures using the axenic medium supplemented with 50 mM MgCl₂. The encystment medium was inoculated with approximately 2 x 10⁵ /ml trophoxoites for culture at 30 °C in a shaking incubator (100 rpm) for 7 days. Cells were enumerated using haemocytometer counting. Microscopic examination showed greater than 90% mature cysts, which were stored at 4 °C for testing within 14 days.

### B. Challenge Test Protocol:

The challenge test assay protocol, as described by R. Hughes and S. Kilvington, Comparison of Hydrogen Peroxide Contact Lens Disinfection Systems and Solutions Against Acanthamoeba Polyphaga, Antimicrob. Agents Chemother. 45: 2038-2043, (2001), is set forth in detail below. The number of surviving organisms was calculated as described by L.J. Reed and H. Muench, A Simple Method for Estimating Fifty Percent Endpoints, Am. J. Hyg., 27: 493-497, (1938). Statistical analysis of the data was by One Way Analysis Of Variance (ANOVA).

### Acanthamoeba Contact Lens Disinfectant Challenge Test Assay

1. Fifty ml polypropylene centrifuge tubes were aged overnight with 15 ml of disinfectant solution, i.e., ReNue ^{™} 02 (Bausch & Lomb, Rochester, New York). The tubes were then drained, the disinfectant solution discarded, and refilled with 10 ml of fresh disinfectant solution for testing.
2. To a flat-bottomed, 96 well microtitre plate having 8 columns labelled "A" through "H", 180 µl of neutralizer, i.e., 0.1 percent Tween^{™} 80 (Baker, Inc., Phillipsburg, NJ) in one-quarter strength Ringer's solution for biguanide based disinfectants, was added to the outer wells of columns A and H. 180 µl of one-quarter strength Ringer's solution was then added to the remaining inner wells of columns B through G.
3. Amoebae were adjusted to 5 x 10⁶ /ml in one-quarter strength Ringer's solution and 100 µl (5 x 10⁵) was added to 10 ml of disinfectant (5 x 10⁴ /ml) or saline control.
4. At time intervals of 0, 1, 2, 3, 4, 6 and 24 hours:
   i. vortexed tubes 5 seconds;
   ii. in quadruplet, removed 20 µl (1000 cells) and added to neutralizer wells for the given time-point (i.e., column A, rows 9-12) and made serial 10-fold dilutions across the microtitre plate (i.e., to column D, rows 9-12); and
   iii. repeated for each time point.
5. One drop of live *E. coli* from 3 ml pastette (25 µl of approximately 5 x 10⁷ bacteria / ml) was then added to each well.
6. The plate was then incubated at 32°C for up to 7 days.
7. The plate was inspected daily for 7 days for the presence of amoebal growth, either trophozoite replication or excystment and trophozoite replication, in the wells.
8. The number of surviving organisms was then estimated at each time interval by Reed and Muench computation and set forth below in Table 3.

**Table 3**

| **Structure-Activity Relationship: Quaternary Ammonium Ester vs** ***Acanthamoeba polyphaga*** | | | |
|---|---|---|---|
| **Comp.No.** | **Amoebae Log Kill (25 ppm) - Time** | **Cysts** | **Trophozoites** |
| 1 | 1 Hr. | 3.30 | |
| | 4 Hr. | 3.30 | |
| | | | |
| 6 | 1 Hr. | 0.46 | |
| | 4 Hr. | 3.54 | |
| | | | |
| 8 | 1 Hr. | 1 | |
| | 4 Hr. | 4 | |
| | | | |
| 9 | 1 Hr. | 3.69 | |
| | 4 Hr. | 4 | |
| | | | |
| 11 | 1 Hr. | 3.69 | |
| | 4 Hr. | 3.69 | |
| | | | |
| 12 | 1 Hr. | 3.54 | |
| | 4 Hr. | 3.54 | |

### Example IV: Sodium Fluorescein Permeability Assay of Compositions 1 and 14

A 0.5 ml cell suspension containing 2x10⁵ cells was seeded in Millicell HA 13 mm inserts (Millipore, Bedford, Massachusetts). The inserts were transferred into 24 well plates containing 0.5 ml of minimum essential medium (MEM) (BioWhittaker, Walkersville, Maryland) per well. The plates were then incubated at 37°C with 5 percent carbon dioxide for six days. Fresh media was added to the wells on days 2 through 6. On day 6 the inserts were used for the permeability assay.

Each insert was gently rinsed three times with 1 ml of Hanks' balanced salt solution (HBSS) using a 10 ml syringe, without a needle. An amount of 0.5 ml of test solution was added to separate inserts that had been placed in a fresh 24 well plate. Triplicate inserts were used for each test solution. The inserts were incubated in a 100 percent humidified chamber at 37 degrees Celsius for twenty minutes. Each series of triplicate samples was handled sequentially to allow exact timing of the treatment and subsequent steps. After incubation, each insert was individually rinsed five times with 1 ml HBSS using a 10 ml syringe without a needle, and then placed in a fresh 24 well plate containing 0.5 ml HBSS in each well. To each insert was added 0.5 ml of sodium fluorescein (3 mg/100 ml in HBSS). The inserts were incubated at room temperature for twenty minutes, removed from the wells, and the amount of sodium fluorescein was measured using a fluorometer at 540 nm excitation and 590 nm emission. Triplicate negative controls (HBSS solution) and positive controls consisting of sodium dodecyl sulphate (SDS) in water were included during these evaluations. Sodium fluorescein permeability assay results for composition numbers 1 and 14 are summarized below in Table 4.

**Table 4**

| **Sodium Fluorescein Permeability Assay Results** | |
|---|---|
| **Sample** | **Fluorescence Units** |
| HBSS (negative control) | 46 |
| SDS (positive control) | 1420 |
| | |
| 100 ppm Compound 1 in BBS | 554 |
| 25 ppm Compound 1 in BBS | 76 |
| 15 ppm Compound 1 in BBS | 77 |
| 10 ppm Compound 1 in BBS | 71 |
| 5 ppm Compound 1 in BBS | 71 |
| | |
| 100 ppm Compound 14 in BBS | 107 |
| 25 ppm Compound 14 in BBS | 65 |
| 15 ppm Compound 14 in BBS | 63 |
| 10 ppm Compound 14 in BBS | 71 |
| 5 ppm Compound 14 in BBS | 59 |

One or more quaternary ammonium ester compounds of the present invention are useful as antimicrobial agents in contact lens care solutions for disinfecting contact lenses. A disinfecting amount of antimicrobial agent is an amount that will at least partially reduce the microorganism population in the formulations employed. Preferably, a disinfecting amount is that which will reduce the microbial burden of representative bacteria by three log orders in four hours and of representative fungi by one log order in one hour. Most preferably, a disinfecting amount is an amount which will eliminate the microbial burden on a contact lens when used according to its regimen for the recommended soaking time (FDA Chemical Disinfection Efficacy Test - July 1985 Contact Lens Solution Draft Guidelines). Typically, such agents are present in concentrations ranging from 0.00001 to 0.5 percent weight/volume (w/v), and more preferably, from 0.00003 to 0.5 percent w/v (25 ppm = 0.0025% w/v).

Solutions of the present invention may likewise include at least one surfactant that has known advantages in terms of cleaning efficacy and comfort. See, for example, U.S. Patent Number 4,820,352 incorporated herein in its entirety by reference. The surfactant should be soluble in the lens care solution, not become turbid, and should be non-irritating to eye tissues.

Likewise, it may be desirable to include one or more water-soluble viscosity agents in the subject solutions. Because of the demulcent effect of viscosity agents, the same have a tendency to enhance the lens wearer's comfort by means of a film on the lens surface cushioning impact against the eye. Suitable viscosity agents include for example but are not limited to cellulose polymers like hydroxyethyl, hydroxypropyl or hydroxypropylmethyl cellulose, guar, hydroxypropylguar, povidone, poly(vinyl alcohol) and the like. Viscosity agents may be employed in amounts ranging from about 0.01 to 4.0 weight percent or less.

Solutions of the present invention may likewise include a buffering system such as those known to those skilled in the art. In a preferred embodiment, the buffer system includes at least one phosphate buffer and at least one borate buffer, which buffering system has a buffering capacity of 0.01 to 0.5 mM, preferably 0.03 to 0.45, of 0.01 N of HCl and 0.01 to 0.3, preferably 0.025 to 0.25, of 0.01 N of NaOH to change the pH one unit. Buffering capacity is measured by a solution of the buffers only.

Solutions of the present invention may likewise include one or more tonicity agents to approximate the osmotic pressure of normal lachrymal fluids which is equivalent to a 0.9 percent solution of sodium chloride or 2.5 percent glycerine solution. Examples of suitable tonicity agents include but are not limited to sodium and potassium chloride, dextrose, mannose, glycerin, calcium and magnesium chloride. These agents are typically used individually in amounts ranging from 0.01 to 2.5 percent w/v and preferably, from 0.2 to 1.5 percent w/v. Preferably, the tonicity agent is employed in an amount to provide a final osmotic value of 200 to 450 mOsm/kg and more preferably between 220 to 350 mOsm/kg, and most preferably between 220 to 320 mOsm/kg.

The pH of lens care solutions of the present invention is preferably maintained within the range of 5.0 to 8.0, more preferably 6.0 to 8.0, most preferably 6.5 to 7.8.

As stated above, contact lenses are disinfected by contacting the lens with the subject aqueous solution. Although this may be accomplished by simply soaking a lens in the subject solution, greater cleaning can be achieved if a few dorps of the solution are initially placed on each side of the lens, and rubbing the lens for a period of time, for example, approximately 20 seconds. The lens can then be subsequently immersed within several milliliters of the subject solution. Preferably, the lens is permitted to soak in the solution for at least four hours. The lenses are then removed from the solution, rinsed with the same or a different solution, for example a preserved isotonic saline solution and then replaced on the eye.

Solutions containing one or more quaternary ammonium ester compounds of the present invention may be formulated into specific contact lens care products for use as customary in the field of ophthalmology. Such products include but are not limited to wetting solutions, soaking solutions, cleaning and conditioning solutions, as well as multipurpose type lens care solutions and in-eye cleaning and conditioning solutions.

While the invention has been described in conjunction with specific examples thereof, this is illustrative only. Accordingly, many alternatives, modifications, and variations will be apparent to those skilled in the art in the light of the foregoing description and it is, therefore, intended to embrace all such alternatives, modifications, and variations as to fall within the spirit and scope of the appended claims.

## Claims

1. Ophthalmic solution comprising a non-irritating antimicrobial amount of a compound of the following: 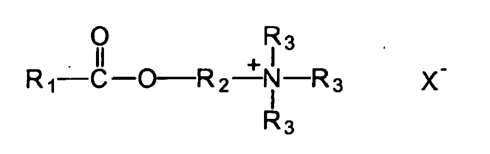 wherein R₁ is a saturated or unsaturated C₁₋₂₄ alkyl, C₁₋₂₄ alkene, C₆₋₂₄ aryl C₆₋₃₆ arylalkyl, C₁₋₂₄ alkyloxy, C₆₋₂₄ haloaryl or C₁₋₂₄ haloalkyl; R₂ is C₁₋₂₄ alkylene, C₆₋₃₆ alkylenearylene, C₆₋₂₄ arylene, C₆₋₃₆ aryloxy or C₁₋₂₄ alkyloxy; the R₃ groups may be the same or different selected from the group consisting of C₁₋₂₄ alkyl, C₆₋₃₆ arylalkyl, C₁₋₂₄ hydroxyalkyl, C₁₋₂₄ alkoxyalkyl C₁₋₂₄ alkoxyalkoxyalkyl and C₁₋₂₄ haloalkyl; and X is a halide, a C₁₋₂₄ alkylsulfate, a C₆₋₃₆ arylsulfate or a pharmaceutically acceptable salt wherein the amount ranges from 0.00001 to 0.5 percent weight/volume; a tonicity adjusting agent to provide an osmolality of 200 to 450 mOsm/kg and water.

2. Solution according to claim 1 wherein the compound is N-myristoyloxypropyl-N-benzyl-N,N-dimethylammonium chloride, N-capryloyloxypropyl-N,N,N-trimethylammonium iodide, N-caproyloxypropyl-N,N,N-trimethylammonium iodide, N-lauroyloxypropyl-N,N,N-trimethylammonium iodide, N-myristoyloxypropyl-N,N,N-trimethylammonium iodide, N-palmitoyloxypropyl-N,N,N-trimethylammonium iodide, N-stearoyloxypropyl-N,N,N-trimethylammonium iodide, N-myristoyloxypropyl-N,N-dimethyl-N-ethylammonium iodide, N-myristoyloxypropyl-N,N-dimethyl-N-propylammonium iodide, N-myristoyloxypropyl-N-butyl-N,N-dimethylammonium iodide, N-myristoyloxypropyl-N,N-dimethyl-N-pentylammonium iodide, N-myristoyloxypropyl-N,N-dimethyl-N-hexylammonium iodide, N-myristoyloxypropyl-N-decyl-N,N-dimethylammonium iodide, N-myristoyloxyethyl-N,N-di(hydroxyethyl)-N-methylammonium iodide or N-myristoyloxyethoxyethyl-N,N,N-trimethylammonium iodide.

3. A method of producing a solution of claim 1 or 2 comprising combining an aminoester with an appropriate weight of a quaternizing agent.

4. A solution according to claims 1 or 2 comprising at least one quaternary ammonium ester in deionized water.

5. The solution of claim 4 wherein said solution includes a buffering system.

6. The solution of claim 4 wherein said solution includes one or more surfactants.

7. The solution of claim 4 wherein said solution includes one or more viscosity agents.

8. Use of the solution of claim 4 for eliminating a microbial burden on said contact lens.

## Patentansprüche

1. Ophthalmologische Lösung umfassend eine nicht reizende, antimikrobielle Menge einer Verbindung entsprechend: 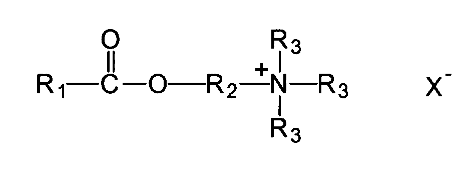 wobei R₁ ein gesättigtes oder ungesättigtes C₁₋₂₄ Alkyl, C₁₋₂₄ Alken, C₆₋₂₄ Aryl C₆₋₃₆ Arylalkyl, C₁₋₂₄ Alkyloxy, C₆₋₂₄ Haloaryl oder C₁₋₂₄ Haloalkyl ist; R₂ ist ein C₁₋₂₄ Alkylen, C₆₋₃₆ Alkylenarylen, C₆₋₂₄ Arylen, C₆₋₃₆ Aryloxy oder C₁₋₂₄ Alkyloxy; die R₃-Gruppen können identisch oder unterschiedlich sein, ausgewählt aus der Gruppe bestehend aus C₁₋₂₄ Alkyl, C₆₋₃₆ Arylalkyl, C₁₋₂₄ Hydroxyalkyl, C₁₋₂₄ Alkoxyalkyl C₁₋₂₄ Alkoxyalkoxyalkyl und C₁₋₂₄ Haloalkyl; und X ist ein Halogenid, ein C₁₋₂₄ Alkylsulphat, ein C₆₋₃₆ Arylsulphat oder ein pharmazeutisch akzeptables Salz, wobei die Menge von 0,00001 bis 0,5 Gew.-%/Volumen reicht; ein tonizitätsregulierender Wirkstoff, um eine Osmolalität von 200 bis 450 mOsm/kg einzustellen, und Wasser.

2. Lösung gemäß Anspruch 1, wobei die Verbindung ausgewählt wird aus N-Myristinoyloxypropyl-N-benzyl-N,N-dimethylammoniumchlorid, N-Capryloyloxypropyl-N,N,N-trimethylammoniumiodid, N-Capronoyloxypropyl-N,N,N-trimethylammoniumiodid, N-Laurinoyloxypropyl-N,N,N-trimethylammoniumiodid, N-Myristinoyloxypropyl-N,N,N-trimethylammoniumiodid, N-Palmitinoyloxypropyl-N,N,N-trimethylammoniumiodid, N-Stearinoyloxypropyl-N,N,N-trimethylammoniumiodid, N-Myristinoyloxypropyl-N,N-dimethyl-N-ethylammoniumiodid, N-Myristinoyloxypropyl-N,N-dimethyl-N-propylammoniumiodid, N-Myristinoyloxypropyl-N-butyl-N,N-dimethylammoniumiodid, N-Myristinoyloxypropyl-N,N-dimethyl-N-pentylammoniumiodid, N-Myristinoyloxypropyl-N,N-dimethyl-N-hexylammoniumiodid, N-Myristinoyloxypropyl-N-decyl-N,N-dimethylammoniumiodid, N-Myristinoyloxyethyl-N,N-di(hydroxyethyl)-N-methylammoniumiodid und N-Myristinoyloxyethoxyethyl-N,N,N-trimethylammoniumiodid.

3. Verfahren zur Herstellung einer Lösung gemäß Anspruch 1 oder 2, umfassend die Vereinigung eines Aminoesters mit einer geeigneten Menge eines quarternierenden Wirkstoffes.

4. Eine Lösung gemäß den Ansprüchen 1 oder 2, umfassend mindestens einen quartemären Ammoniumester in deionisiertem Wasser.

5. Die Lösung gemäß Anspruch 4, wobei die genannte Lösung ein Puffersystem mit einschließt.

6. Die Lösung gemäß Anspruch 4, wobei die genannte Lösung ein oder mehrere Tenside mit einschließt.

7. Die Lösung gemäß Anspruch 4, wobei die genannte Lösung ein oder mehrere viskositätsregulierende Wirkstoffe mit einschließt.

8. Verwendung der Lösung gemäß Anspruch 4 zur Entfernung einer mikrobiellen Belastung auf genannter Kontaktlinse.

## Revendications

1. Solution ophtalmique comprenant une quantité antimicrobienne non irritante d'un composé de formule suivante : 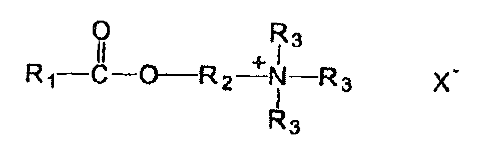 où R₁ est un groupe alkyle en C_{1 à 24}, alcène en C_{1 à 24}, aryle en C_{6 à 24}, arylalkyle en C_{6 à 36}, alkyloxy en C_{1 à 24}, halogénoaryle en C_{6 à 24} ou halogénoalkyle en C_{1 à 24} saturé ou insaturé ; R₂ est un groupe alkylène en C_{1 à 24}, alkylènearylène en C_{6 à 36}, arylène en C_{6 à 24}, aryloxy en C_{6 à 36} ou alkyloxy en C_{1 à 24} ; les groupes R₃ peuvent être identiques ou différents et sont choisis dans le groupe constitué des groupes alkyle en C_{1 à 24}, arylalkyle en C_{6 à 36}, hydroxyalkyle en C_{1 à 24}, alcoxyalkyle en C_{1 à 24}, alcoxyalcoxyalkyle en C_{1 à 24} et halogénoalkyle en C_{1 à 24} ; et X est un halogénure, un alkylsulfate en C_{1 à 24}, un arylsulfate en C_{6 à 36} ou un sel pharmaceutiquement acceptable où la quantité est dans la gamme de 0,00001 à 0,5 pour cent en poids/volume ; un agent d'ajustement de tonicité pour fournir une osmolalité de 200 à 450 mOsm/kg et de l'eau.

2. Solution selon la revendication 1, dans laquelle le composé est le chlorure de N-myristoyloxypropyl-N-benzyl-N,N-diméthylammonium, l'iodure de N-capryloyloxypropyl-N,N,N-triméthylammonium, l'iodure de N-caproyloxypropyl-N,N,N-triméthylammonium, l'iodure de N-lauroyloxypropyl-N,N,N-triméthylammonium, l'iodure de N-myristoyloxypropyl-N,N,N-triméthylammonium, l'iodure de N-palmitoyloxypropyl-N,N,N-triméthylammonium, l'iodure de N-stéaroyloxypropyl-N,N,N-triméthylammonium, l'iodure de N-myristoyloxypropyl-N,N-diméthyl-N-éthylammonium, l'iodure de N-myristoyloxypropyl-N,N-diméthyl-N-propylammonium, l'iodure de N-myristoyloxypropyl-N-butyl-N,N-diméthylammonium, l'iodure de N-myristoyloxypropyl-N,N-diméthyl-N-pentylammonium, l'iodure de N-myristoyloxypropyl-N,N-diméthyl-N-hexylammonium, l'iodure de N-myristoyloxypropyl-N-décyl-N-diméthylammonium, l'iodure de N-myristoyloxyéthyl-N,N-di(hydroxyéthyl)-N-méthylammonium ou l'iodure de N-myristoyloxyéthoxyéthyl-N,N,N-triméthylammonium.

3. Procédé de production d'une solution selon la revendication 1 ou 2, comprenant l'étape consistant à mélanger un aminoester avec un poids approprié d'un agent de quaternisation.

4. Solution selon les revendications 1 ou 2, comprenant au moins un ester d'ammonium quaternaire dans de l'eau désionisée.

5. Solution selon la revendication 4, dans laquelle ladite solution comprend un système tampon.

6. Solution selon la revendication 4, dans laquelle ladite solution comprend un ou plusieurs agents tensioactifs.

7. Solution selon la revendication 4, dans laquelle ladite solution comprend un ou plusieurs agents de viscosité.

8. Utilisation de la solution selon la revendication 4, pour éliminer une charge microbienne sur ladite lentille de contact.
